# EUROPÄISCHE PATENTSCHRIFT

(11) **EP 3 568 123 B1**
(45) Veröffentlichungstag und Bekanntmachung des Hinweises auf die Patenterteilung: **21.07.2021**
(21) Anmeldenummer: 17838126.5
(22) Anmeldetag: 20.12.2017
(51) Int. Cl.: A61K 8/02, A61K 8/06, A61K 8/14, A61K 8/73, A61Q 19/00, A61K 9/107, A61K 9/113, A61K 9/127, A61K 9/51, A61K 31/7036

(54) **EINBRINGEN VON SCHWER UND/ODER NICHT WASSERLÖSLICHEN WIRKSTOFFEN MITTELS AUF LIPIDEN BASIERENDEN NANOPARTIKELN/VESIKELN IN EIN HYDROPHILES AUS CELLULOSE BESTEHENDES DREIDIMENSIONALES NETZWERK**
INTRODUCTION OF HARDLY SOLUBLE AND/OR WATERSOLUBLE ACTIVE AGENTS WITH LIPID BASED NANOPARTICLES/NANOVESICLES INTO A HYDROPHILIC THREEDIMENSIONAL NETWORK OF CELLULOSE
INTRODUCTION DE SUBSTANCES ACTIVES FAIBLEMENT SOLUBLES ET/OU NON SOLUBLES DANS L'EAU AU MOYEN DE NANOPARTICULES/VÉSICULES À BASE DE LIPIDES DANS UN RESEAU TRIDIMENSIONNEL HYDROPHILE DE CELLULOSE

(30) Priorität: 16.01.2017 DE 102017000368
(43) Veröffentlichungstag der Anmeldung: 20.11.2019
(73) Patentinhaber: Blume, Gabriele, 36396 Steinau an der Strasse (DE)
(72) Erfinder: FISCHER, Dagmar, 07743 Jena (DE); ALKHATIB, Yaser, 07743 Jena (DE); BLUME, Gabriele, D-36396 Steinau an der Strasse (DE)
(74) Vertreter: advotec.
(86) Internationale Anmeldenummer: PCT/EP2017/083778
(87) Internationale Veröffentlichungsnummer: WO 2018/130390

(56) Entgegenhaltungen:
- WO-A1-2015/196042
- WO-A2-2007/027849
- WO-A2-2013/060321
- DE-A1-102013 001 002
- US-A1- 2013 330 379
- MIKULCOVÁ VERONIKA ET AL: "On the preparation and antibacterial activity of emulsions stabilized with nanocellulose particles", FOOD HYDROCOLLOIDS, ELSEVIER BV, NL, Bd. 61, 25. Juni 2016 (2016-06-25), Seiten 780-792, XP029683523, ISSN: 0268-005X, DOI: 10.1016/J.FOODHYD.2016.06.031

## Beschreibung

Cellulose ist ein Polysaccharid, das den Hauptbestandteil der pflanzlichen Zellwand bildet. Dieser pflanzliche Rohstoff findet von jeher seinen Einsatz, z.B. in der Papierindustrie sowie in der Herstellung von Textilien. Vor ca. 70 Jahren wurde durch die Kultivierung spezieller Bakterien durch Hestrin und Schramm auch die Herstellung von bakterieller Nanocellulose (BNC) ermöglicht (H. Hestrin und H. Schramm; Biochem. J. 58 (1954) 345-352). Von da an trat die BNC ihren Siegeszug in den unterschiedlichsten Bereichen an, gut nachzulesen im Artikel "Bakterielle Cellulose: Produktion und Applikation; ein Rückblick über 10 Jahre" (A. Jozala et al; Appl.Microbiol. Biotechn. 100 (2016) 2063-2072).

Die BNC besteht aus einem dreidimensionalen Netzwerk aus Cellulose-Nanofasern, die einen Durchmesser von ca. 20 bis 100 nm aufweisen und damit 100-mal dünner sind als die pflanzliche Cellulose. Vorwiegend gramnegative, aerobe Bakterien der Spezies Komagateibacter xylinus, aber auch viele andere Essigsäurebakterien synthetisieren aus niedermolekularen Zuckern durch Polymerisation zunächst die Celluloseketten, die dann über mehrere Schritte ein hochgequollenes dreidimensionales extrem feines Netzwerk bilden. Technologisch betrachtet handelt es sich um ein formstabiles Hydrogel oder Hydropolymer, bestehend aus etwa 1% Cellulose und 99% Wasser (D. Fischer und D. Kralisch, Deutsche Apotheker Zeitung Nr. 36, 56-62, 2012). Diese Cellulose entspricht chemisch pflanzlicher Cellulose, weist allerdings ca. 100fach kleinere Faserdurchmesser auf, ist wesentlich reiner herstellbar (ohne Begleitstoffe wie z.B. - Lignin), gut zu sterilisieren, biokompatibel und zeigt deutlich höhere mechanische Stabilität.

Aufgrund dieser Eigenschaften wird die BNC folgendermaßen beschrieben: "Vielseitiges und erneuerbares Polymer für medizinische Anwendungen" (H. Gomes de Oiliveira Barud et. al; Carbohydrate Polymers 153 (2016) 406-420); "Vielseitig, hochentwickeltes Material" (N. Shah et al.; Carbohydrate Polymers 98 (2013) 1585-1598); "Vielversprechendes natürliches Polymer für die Wundbehandlung" (L. Fu et al.; Carbohydrate Polymers 92 (2013) 1432-1442). Ihre gute Hautverträglichkeit und die feuchtigkeitsspendenden Eigenschaften lassen eine umfangreiche dermale Anwendung in der Kosmetik und Pharmazie/Medizin zu. Beispiele von u.a. auf dem Markt befindlichen Produkten in der Kosmetik als feuchtigkeitsspendende Gesichtsmasken sind "epi novelle+ basic" (Jenacell, D), "Nanomasque" (fzmb GmbH), "Epitrans⁺" (Beaumetics, D), oder "CELMAT" (Bowil biotech, PL) und im Rahmen der medizinischen Wundbehandlung "Suprasorb X" (Lohmann & Rauscher, D), "Cuticell Epigraft" (BSN medical GmbH, D) oder "Dermafill" (Fibrocel, BR).

In mehreren Patenten und Artikeln wird auf die Herstellung der BNC aus unterschiedlichen Bakterienarten sowie auf die chemische Modifikation der Cellulose eingegangen. DE 10 2013 001 002 A1 beschreibt das Verfahren und die Vorrichtung zur Herstellung von biotechnologisch gewonnener Nanocellulose in flächiger Form. EP 2633033 A2 umfasst die Beschreibung eines Hydrogels basierend auf bakteriellen Cellulose-Nanofasern und/oder deren mechanische zerfaserten Derivate. Eine sehr aktuelle Patentmeldung beschreibt die Herstellung eines multi-phasischen Biomaterials bestehend aus verschiedenen BNC-Layern, die durch unterschiedliche Bakterienstämme synthetisiert werden (WO 2016/113 400 A1). Hernán Charreau und Kollegen beschreiben 2013 ausführlich das wachsende Interesse am Einsatz von Nanocellulose durch die Zunahme an wissenschaftlichen Artikeln und der steigenden Zahl an Patenten meist die Produktion und Applikation von Cellulose-Nanofasern und BNC betreffend (H. Charreau et al.; Recent Patents on Nanotechnology 7 (2013) 56-80).

In der letzten Zeit taucht immer häufiger der Begriff "Bakterielle Nanocellulose als potentielles Wirkstoff-Trägersystem" auf. Übersichtsartikel zeigen die ersten Ansätze von BNC als Arzneistoffträgersystem, besagen aber auch, dass noch umfangreiche Forschung und Entwicklung diesbezüglich betrieben werden muss (A. Silvestre et al.; Expert Opinion 11 (2014) 1113-1124; M. Abdeer et al.; J Pharmacy & Pharmacology (2014) 3-15; M. Jorfi und E. Foster; Applied Polymer 41719 (2015) 1-19).

So ermöglichen sehr aufwendige biosynthetische oder chemische Modifikationen der Cellulosefasern während der Herstellung des Netzwerks eine neuartige nanostrukturelle Matrix für eine Vielzahl von Anwendungen (in situ). Diese Änderungen in der Morphologie der BNC sind aber mit Vorsicht zu betrachten: So müssen aufwendige und kostenintensive neue klinische Untersuchungen mit der modifizierten Cellulose-Matrix durchgeführt werden (u.a. Biokompatibilität, nicht allergene, nicht irritative und nicht toxische Eigenschaften). Folgende Artikel beinhalten einen umfangreichen Überblick hinsichtlich der Modifikation der bakteriellen Cellulose (W. Hu et al. Carbohydrates Polymers 101 (2014) 1043-1060 und I. Sulaeva et al.; Biotechnology Advances 33 (2015) 1547-1571).

Dagegen ist die Wirkstoffbeladung der BNC nach deren kompletten Herstellung wesentlich einfacher und preisgünstiger ("post synthesis loading"). Dies erfolgt unter anderem, indem man die native BNC in einer wirkstoffhaltigen Lösung / Dispersion tränkt oder aber die partiell getrocknete oder lyophilisierte Cellulose in Lösungen mit den aktiven Substanzen requellen lässt.

Solche Beschreibungen der Herstellung von BNC-Wirkstoffträgersystemen werden z.B. in einigen Patentanmeldungen angeben, aber ohne auf Details einer reproduzierbaren Durchführung einzugehen (z.B. US 2007/0053960 A1 oder US 2013/0330417 A1 oder US 2014/0213764 A1).

Im medizinischen Bereich speziell für die Wundbehandlung befinden sich erst wenige Produkte auf dem Markt. Da die BNC selbst keine anti-mikrobielle Aktivität aufweist, werden deshalb keimtötende Stoffe beigefügt wie z.B. Silber (US Patent 8,293,267 B2) oder das antiseptisch wirkende Polyhexa-methylenbiguanid (Suprasorb X+PHMB der Firma Lohmann & Rauscher).

Gerade das Interesse der Anwendung von BNC als Wirkstoffträgersystem in der Kosmetik und Pharmazie/Medizin steigt, was man anhand der Zunahme an wissenschaftlichen Artikeln sehen kann. In Tabelle 1 sind Veröffentlichungen zusammengefasst, die sich mit der "post synthesis"- Beladung von BNC mit Arzneistoffen befassen.

Bei eingehender Betrachtung der Veröffentlichungen ist auffallend, dass so gut wie keine lipophilen Stoffe/Arzneimittel in die Cellulosematerialien eingearbeitet worden sind. Der extrem hohe Wasseranteil des BNC-Hydrogels beinhaltet hohe Anforderungen, einen fettlöslichen Stoff homogen in dem gesamten dreidimensionalen Netzwerk zu verteilen sowie kontrolliert freizusetzen.

Es wurde u.a. versucht, "post synthesis" durch eine aufwendige Modifikation der BNC-Oberfläche - Acetylierung - die Hydrophobie der Matrix zu verbessern. Dies sollte eine erhöhte Bindung der lipophilen Wirkstoffe auf der umfangreichen Oberfläche des Netzwerkes ermöglichen (DY. Kim et al.; Cellulose 9 (2002) 361-367).

Ohne Modifikation wurde z.B. Ibuprofen als ethanolische Lösung direkt in eine vorbereitete nur auf Ethanol basierende BNC eingebracht (E. Trovatti et al.; Int. J.Pharm. 435 (2012) 83-87). Das Einbringen von Alkohol kann sowohl die Struktur als auch die mechanischen Eigenschaften und Konsistenz der BNC gravierend beeinflussen. Zwei weitere lipophile Wirkstoffe wurden zum Beladen der BNC mit amphiphilen Block-Polymeren versetzt. Im wässrigen Medium lagern sich diese Substanzen spontan zu Aggregaten = Mizellen zusammen und können somit ohne "große" Schwierigkeiten in das BNC-Hydrogel eingearbeitet werden. Die Mizellen bestanden aus Retinol und Poly(ethylene oxide)-b-poly(caprolactone) oder Octenidine und Poloxameren. Die hier eingesetzten Blockcopolymere besitzen ein sehr hohes Molekulargewicht und können eine schnelle Degradation in physiologischer Umgebung aufweisen. So wurde z.B. das Retinol schon in der BNC freigesetzt, welches zu einer Präzipitation des Vitamins im Netzwerk führte (Y. Numata et al.; Int.J.Pharm. 486 (2015) 217-225). Andererseits lassen sich verzögerte Freisetzungen bis zu einer Woche, wie z.B. im Falle des Poloxamer/Octenidin-Systems, erreichen.

Hieraus ergab sich die Aufgabe, geeignete Lipidzusammensetzungen zu finden, die stabile Partikel/Vesikel zur Verkapselung der lipophilen Wirkstoffe bilden, zugleich aber in wässriger Lösung, d.h. Dispersion vorliegen und sich somit einfach in das hydrophile Netzwerk der Cellulose einarbeiten lassen.

Dermale Trägersysteme, wie z.B. Mizellen, Liposomen, Niosomen, Nanoemulsionen (Öl-in-Wasser) oder multiple Nanoemulsionen (Wasser-in-Öl-in-Wasser) sowie Lipidnanopartikel, werden als Wirkstoffträger für schwerlösliche bzw. lipophile Wirkstoffe sowohl in der Kosmetik als auch in der Pharmazie/Medizin eingesetzt (B.W. Barry; Eur.J.Pharm.Sci. 14 (2001) 101-114; P.L. Honeywell-Nguyen and J.A. Bouwstra; Drug Discovery Today 2 (2005) 67-74). Diese Systeme sind kleinste Partikel im Nanometerbereich und werden aus mindestens einem lipophilen langkettigen fettsäurehaltigen Emulgator gebildet. Diese Emulgatoren können z.B. nur aus sich selbst mit dem Wirkstoff die Mizellen bilden, ein Öltröpfchen umschließen (Nanosomen, Emulsionen), ein Wasser-im-Öltröpfchen umgeben (multiple Nanoemulsion), als auch als Doppelmembran einen wässrigen Innenkern umschließen (z.B. Liposomen, Transfersomen, Ethosomen , Niosomen) oder eine "homogene" aus verschiedenen Lipiden bestehende Matrix formen (Lipidnanopartikel). All diese Partikel/Vesikel liegen als wässrige Dispersion vor, wobei die Teilchen optisch einzeln nicht sichtbar sind. Sie werden u.a. in der Pharmazie und Kosmetik eingesetzt, um sehr empfindliche Stoffe zu stabilisieren (z.B. Vitamine) und Wirkstoffe in die Haut zu bringen. Das ermöglicht eine erhöhte Bioverfügbarkeit der Wirkstoffe an der Stelle wo sie benötigt werden. Solche Trägersysteme und ihre Eigenschaften sind in den folgenden Übersichtsartikeln beschrieben: P. Honeywell-Nguyen und J. Bouwstra; Drug Delivery Today 2 (2005) 67-74; B. Barry; Eur. J. Pharm. Sci. 14 (2001) 101-114; D. Prasanthi und P. Lakshmi; Asian J. Pharmaceutical + Clinical Research 5 (2012) 18-25; M. Gangwar et al.; Asian Pacific J. of Tropical Biomedicine (2012) 1176-1188; V. Patravale und S. Mandawgade; Int.J. Cosm. Sci. 30 (2008) 19-33).

Stabile Liposomen bestehen aus Phospholipiden, speziell dem Phosphatidylcholin (>70%), welches die Doppelmembran der Vesikel bildet, die einen wässerigen inneren Kern umschließt. Natürliche Phospholipide werden aus dem Hühnerei und pflanzliche vorwiegend aus Soja und der Sonnenblume gewonnen, können aber auch synthetisch gerade für pharmazeutische Anwendungen hergestellt werden.

Niosomen (non-ionic surfactant) sind aufgebaut wie die Liposomen, nur dass hier u.a. nicht ionische Detergentien zu Bildung der Doppelmembran eingesetzt werden (G. Kumar und P. Rajeshwarrao; Acta Pharmaceutica Sinica B1 (2011) 208-219). Vorwiegend sind das Ester/Ether-Verbindungen der Fettsäure (z.B. Palmitin- Stearin- oder Ölsäure) mit folgenden hydrophilen Substanzen (z.B. Sorbitol, Sucrose, Polyethylenglycole oder Polyglycerole) - als stabilisierende Zusätze der Vesikel werden meistens auch noch Cholesterol oder andere pflanzliche Sterole beigefügt.

Lipid-Nanopartikel bestehen aus einem oder mehreren unterschiedlichen Lipiden, die eine durchgehende Matrix bilden. Mindestens eine der Lipidkomponenten liegt hierbei bei Raumtemperatur in fester Form vor (solid), während die anderen flüssig sind oder ein Öl sein können. Gerade in diesem flüssigen Anteil lassen sich lipophile Wirkstoffe gut einbetten (J. Pardeike et al.; Int. J. Pharm. 366 (2009) 170-184).

Unter einer Emulsion versteht man ein fein verteiltes Gemisch zweier normalerweise nicht mischbarer Flüssigkeiten ohne sichtbare Entmischung (Wikipedia). Bei einer Öl-in-Wasser-Emulsion (O/W) handelt es sich um das Einbringen einer lipophilen, öligen Phase in eine wässerige, deren Phasentrennung durch geeignete Emulgatoren verhindert wird. Diese Emulgatoren umschließen dann das Öltröpfchen als eine Membran und unterbinden somit die Freisetzung gelöster lipophiler Wirkstoffe in die wässrige Umgebung. Man unterscheidet in der Kosmetik und Pharmazie zwischen Nanoemulsionen (> 80 nm und milchig-opaleszent, hoher Anteil der lipophilen Phase bei einem geringen Anteil an Emulgatoren) und Mikroemulsionen (< 100 nm und transparent, geringer Anteil der lipophilen Phase bei einem hohen Anteil an Emulgatoren).

Eine multiple Emulsion (Wasser-in-Öl-in-Wasser) unterscheidet sich dadurch, dass sich im Inneren der Vesikel noch ein Wasseranteil in dem Öltröpfchen befindet. Somit können in diesem Partikel neben den lipophilen Wirkstoffen auch hydrophile oder amphiphile Wirkstoffe eingebracht werden. Nanovesikel, die als dermale penetrierende Wirkstoffträger fungieren sollen, müssen eine sehr flexible Membran und eine kleine Teilchengröße aufweisen.

Folgende Emulgatoren zeichnen sich für die Herstellung solcher Emulsionen aus: Ester und/oder Ether-Verbindungen von langkettigen Fettsäuren (größer oder gleich C14). Die Fettsäuren können u.a. mit folgenden Stoffen verbunden werden: n-Polyethylenglycol, n-Polyglycerol, Zuckern, Zucker-alkohole, kurzkettigen Alkoholen, kurzkettigen Carbon- und Dicarbonsäuren und Aminosäuren.

In Tabelle 2 sind einige in der Pharmazie zugelassene Emulgatoren aufgezeigt, die für eine bessere Penetrationseigenschaft der Trägersysteme in die Haut geeignet sind. Deren Fettsäuren sind ungesättigt bzw. mehrfach-ungesättigt, was die Flexibilität der Partikel erhöht. Ebenso eignen sich ähnliche oder gleiche wie die in Tabelle 2 beschriebene Emulgatoren; nur sind deren Fettsäuren gesättigt. Dies erhöht die Stabilität dieser Partikel, behindert aber deren Aufnahme in die Haut. Dadurch können sie mit ihren verkapselten Wirkstoffen auf der Hautoberfläche aktiv werden.

Somit werden gekonnt je nach Art der verwendeten Trägersysteme die gewünschten Anwendungen der Wirkstoffe praktikabel umgesetzt.

Noch nie zuvor wurde das Einbringen solcher auf Lipiden basierenden dermalen Trägersysteme, in denen mindestens ein schwer wasserlöslicher oder ein lipophiler Wirkstoff bzw. ein Öl nanopartikulär verkapselt ist, in ein aus Cellulose bestehendes dreidimensionales Netzwerk beschrieben.

In dieser Anmeldung wird besonders die Beladung der Cellulose mit zwei neuartigen Trägersystemen hervorgehoben, den "Sophi-Hydro-Tops" und "Sophi-Lipo-Tops". Diese Nanopartikel zeichnen sich durch eine hohe Verkapselungseffizienz für schwerlösliche bzw. lipophile Wirkstoffe aus und zeigen zudem eine hohe Bioverfügbarkeit der Aktiva, verbunden mit guten Penetrationseigenschaften in die Haut (www.sopharcos.de).

Bei den Lipo-Tops handelt es sich um eine Öl-in-Wasser-Nanoemulsion, die sich durch sehr hohe Einschlussraten bis zu 20% an lipophilen Wirkstoffen und/oder Ölen auszeichnet (EP2658610 B1). Die Hydro-Tops sind eine Wasser-in-Öl-in-Wasser-Nanoemulsion, die eine geringere Verkapselungs-effizienz für lipophile Wirkstoffe aufweist (bis ca. 2%). Das Innere dieser Nanopartikel beinhaltet Wassertröpfchen, die von einer Ölphase umgeben sind, wobei die Partikel dann als wässerige Dispersion vorliegen. Somit können in diese Nanopartikel sowohl lipophile und schwer wasserlösliche, amphiphile als auch hydrophile Substanzen eingeschlossen werden (EP 2552413 B1). Beide Systeme beruhen auf dem membranbildenden Emulgator Glyceryl Citrate/Lactate/Linoleate/Oleate (Imwitor 375).

Ferner können natürlich auch die altbewährenden Liposomen als Trägersystem für die Verkapselung von lipophilen Wirkstoffen eingesetzt werden.

Die Zusammensetzung der in dieser Anmeldung vorwiegend eingesetzten Vesikel ist in der Tabelle 3 aufgeführt.

In dieser Anmeldung wird zum ersten Mal das Einbringen solcher auf Lipiden basierenden nanopartikulären Wirkstoffträgersysteme in ein aus Cellulose bestehendes dreidimensionales Netzwerk (vorwiegend BNC) beschrieben. Diese Beladung der BNC mit den Nanopartikeln kann mit unterschiedlichen Methoden "post synthesis" erfolgen.
1) Beladen von teilweise oder vollständig entwässerten Cellulose-Vliesen oder der gefriergetrockneten Variante durch **"Requellen".**

Diese Beladung ist die am häufigsten verwendete Anwendung, da ein hohes Einbringen des in wässeriger Lösung befindlichen Wirkstoffs in die BNC erfolgt (u.a. US 2007/0053960 A1 oder US 2013/0330417 A1 oder US 2014/0213764 A1). Die teilweise Trocknung kann durch Handpressung, hydraulisch oder aber durch das An-/Austrocknen unter Luft/Wärme erfolgen. Die Gefriertrocknung / Lyophilisation ergibt dagegen eine vollständig entwässerte BNC (gute Übersicht ist in der Dissertation von Astrid Müller gegeben, Friedrich Schiller Universität 2016, S.24-28). Negativ dagegen sind die strukturellen Veränderungen des Netzwerks hervorgerufen durch die unterschiedlichen Trocknungsmethoden, die ein reversibles Requellen der Cellulose beeinflussen. Das gilt ebenso für eine kritische Punkt-Trocknung der BNC. Im feuchten Zustand ist die BNC ein Hydrogel, welches unter Lufttrocknung einen Strukturkollaps erleidet (C. Clasen et al.; Macromol. Sym. 244 (2006) 48-58). Im Hinblick auf eine bestmögliche Requellung der BNC wird ein hoher Strukturerhalt bei der Trocknung angestrebt. Dabei hat sich die Gefriertrocknung als am besten herauskristallisiert (D. Klemm et al.; Prog. Polym Sci 26 (2001) 1561-1603). In der Patentanmeldung WO 2013/060321 A3 wird sehr darauf eingegangen, wie cellulosehaltige Materialien zu trocknen sind, um sie danach wieder möglichst in ursprünglicher Struktur und Konsistenz requellen zu lassen (sowohl luftgetrocknet als auch gefriergetrocknet).

Hierbei wird zum Requellen/Beladung des Cellulosematerials mindestens wieder die Menge an wässeriger Lösung eingesetzt, der zuvor durch die Trocknung entzogen wurde.

Folgende Aspekte legen eine solche Beladung dar: das gefriergetrocknete Material ist einfach zu verpacken und somit stabil aufzubewahren. Vor jeder dermalen Applikation lässt sich dann die BNC "einfach" mit dem speziell angepassten wirkstoffbeladenen Nanopartikeln beladen (on demand). Außerdem ist die trockene Variante der BNC als Wirkstoffträger, z.B. bei der Wundbehandlung, besser anzuwenden, wenn weitere Auflagen/Bandagen darüber angebracht werden müssen.
2) Beladen nativer "feuchter" Cellulose-Vliese mittels Sorptionsmethode **("Schüttelmethode"**).

Hierbei wird die unbehandelte native Cellulose mit einem Überschuss einer wässerigen Dispersion inkubiert, die die mit dem wirkstoffbeladenden lipophilen Nanopartikel enthält. Diese über Diffusion und/oder Quellung hervorgerufene Beladung erfolgt unter einem ständigen leichten Schütteln (z.B. 70 rpm oder 200 rpm) über längere Zeiträume (z.B. 1-72h). Unterschiede in der Beladung von feuchter (never-dried=nd) oder gefriergetrockneter (freeze-dried=fd) BNC mit Albumin zeigten eine deutlich höhere Beladungseffizienz für die nd BNC (ca. 25%) beruhend auf den nicht veränderten Strukturen des Netzwerks. Darauf beruhen auch Unterschiede in den absolut freigesetzten Wirkstoffmengen (A. Müller et al.; J. Pharm Sci. 102 (2013) 579-592). Das Einbringen der Nanopartikel mit ihrem verkapselten Wirkstoff und auch deren spätere Freisetzung kann somit schon durch die Trocknungsart des Cellulosenetzwerks beeinflusst werden.
3) Beladen nativer Cellulose-Vliese mittels Sorptionsmethode **(Vortexmethode** = **"high speed**").

Mit dieser Technik werden die Prozesszeiten der Beladung des nativen Cellulose-Hydrogels mit den Nanopartikeln deutlich verkürzt, womit eine deutlich effizientere und kostengünstigere industrielle Herstellung der BNC als Wirkstoffträger gewährleistet wird. Eine lange Beladungsdauer kann sich gegebenenfalls auch negativ auf empfindliche sensitive Wirkstoffe auswirken (z.B. Stabilität von Vitaminen verringern). Wie zuvor beschrieben wird die native BNC in die wässerige Dispersion gegeben, danach aber mittels Vortexen (über turbulente dynamische Strömung) nur für kurze Zeit gründlich durchmischt (5-60 Minuten). Hiermit kann eine vergleichbare Beladungskapazität wie bei der konventionell angewendeten Schüttelmethode erzielt werden. Die Freisetzung der unter "high speed" beladenen Wirkstoffe erfolgt in den ersten Stunden wie die der unter der Schüttelmethode eingebrachten Wirkstoffe. Die zweite Phase der Freisetzung ist dagegen geringer und langsamer mit der Vortex-Variante (A. Müller et al.; Carbohydrate Polymers 106 (2014) 410-413).
4) Beladen nativer Cellulose-Vliese mittels **"Einbringen der Nanopartikel/Wirkstoffs in den Kern des BNC-Materials".**

Diese Methode erfordert den aufwendigsten Einsatz. Hierbei wird in den Mittelpunkt des dreidimensionalen Netzwerks die Nanopartikeldispersion eingebracht z.B. mit einer Spritze injiziert. Über die Zeit können sich die mit wirkstoffbeladenen Partikel über das gesamte Netzwerk mittels Diffusion verteilen. Dieser Einsatz erweist sich eher geeignet für größere Partikel oder für die festeren Lipid-Nanopartikel.

Je nach Anwendung der Methode des Einbringens der unterschiedlichen wirkstoffbeladenen Nanopartikel (Requellen, Sorption schütteln oder vortexen und Kernbeladung) in die unterschiedlichen BNC-Varianten (getrocknet oder nicht getrocknet) kann eine gezielte Steuerung sowohl der Beladungsmenge mit dem Wirkstoff in der BNC und deren anschließenden Freisetzung erfolgen. So kann je nach Anwendung zwischen "feuchter" BNC oder eher "trockener" BNC unterschieden werden, sowie zwischen einem wirkstoffhaltigem Depot mit langsamer Freisetzung des Arzneimittels z.B. in medizinischen Produkten oder sofort einsetzender Wirkstoffabgabe z.B. in der Kosmetik.

Das erfindungsgemäße Herstellungsverfahren wird in Anspruch 1 beschrieben.

Die erfindungsmäßige Zubereitung wird in Anspruch 6 definiert und setzt sich zusammen aus dem dreidimensionalen Netzwerk bestehend aus Cellulosefasern, den Nanopartikeln und den darin befindlichen schwer wasserlöslichen und/oder lipophilen nanopartikulären Wirkstoffen. Hier ist besonders auf die bakterielle Nano-cellulose einzugehen, die schon erste Anwendungen in der Kosmetik und Pharmazie / Medizin hat.

Medizinische und kosmetische Verwendungen der erfindungsgemäßen Zusammensetzung sind Gegenstand der Ansprüche 10, 11 und 12.

In der Kosmetik werden solche Cellulose-Vliese (pflanzlich oder bakteriell hergestellt) eingesetzt, die sich wie eine zweite Haut auf dem Körper anschmiegen, das besonders im Skin Care Bereich (u.a. Augen und Stirnbereich) aber auch zur Körperpflege, selbst an Händen und Füßen. Bevorzugt sollen in diese Vliese Nanopartikel mit Wirkstoffen natürlichen Ursprungs (bevorzugt pflanzlich) eingebracht werden. In der Kosmetik werden besonders tierische aber auch synthetische Wirkstoffe ungern eingesetzt. Ein Auszug der kosmetisch und/oder dermatologisch wirksamen lipophilen Substanzen, die eingesetzt werden können, kann bestehen aus: Algen- und Pflanzenextrakten (z.B. Phlorogine von Biotechmarine, ein Algenextrakt zur Behandlung von fettiger Haut oder z.B. Incromega V3 von Croda, ein Pflanzenextrakt von Echiomega); Anti-Cellulite wirksame Substanzen (z.B: CLA, konjugierte Linolsäure); Anti-Elastase und Anti-Collagenase wirksame Stoffe (z.B. ungesättigte Fettsäuren wie Ölsäure oder EPA= Eicosapentaensäure); anti-inflammatorisch wirksame Stoffe (marinen oder pflanzlichen Ursprungs); Antioxidantien (z.B. Salbei Extrakt von Flavex, Liponsäure und deren Derivate); Ceramide (z.B. verschiedene Ceramide der Firma Cosmoferm); hautberuhigende und hautglättende Wirkstoffe (z.B. Bisabolol); Moisturiser (z.B. Glycerolmonoisostearate, Sucrose Polysoyate); Flavonoide (zu den Flavonoiden gehören Flavanole, Flavanone, Anthocyanidine, Flavone und Flavonole wie z.B. Sinensetin oder Polyphenole wie die im Grüntee oder Trauben); Phytosterole (wie Sitosterol aus Maisfaseröl); Radikalfänger (z.B. Ubichinol-Derivate wie Coenzym Q10); Saponine (z.B. vom Ginseng, Süßholzwurzel und Rosskastanie); sauerstoffbindende Substanzen (z.B. Perfluordekalin); sebumreduzierende Substanzen (z.B. 10-Hydroxydecansäure); Stoffe, die die Durchblutung und damit die Versorgung der Haut fördern (z.B. Nikotinsäureester); Terpene (kosmetisch und dermatologisch relevante Terpene sind aufgeführt in der Pharmazeutischen Zeitung Heft 22; 2006 von Sebastian Jäger u.a.); Vitamine (Retinol und Derivate, Vitamin E und Derivate wie Tocotrienole oder Carotine und Carotinoide wie Lycopene, Lutein oder Fucoxanthin, Vitamin D und Derivate); und lipophile Peptide als Anti-Ageing Produkt (z.B. Ester von 2-10 Aminosäuren, die an eine Fettsäure (C14-C18) gebunden sind).

Alle diese Wirkstoffe sollen die Hautalterung und/oder das Photoageing (durch UV- bzw. Umweltbelastung, Effekte hervorgerufen durch Pollution) verhindern bzw. inhibieren; die Synthese von dermalen und epidermalen relevanten Makromolekülen (z.B. Kollagen, Elastin) stimulieren bzw. deren Degradation verhindern; die Proliferation von Fibroblasten und Keratinozyten anregen und somit den Schutz und die Erhaltung einer gesunden Haut bewirken. Im Endeffekt betrifft dies alle Wirkstoffe, die eine Aufrechterhaltung der gesunden Haut erwirken bzw. deren Regenerierung wieder zu einer physiologisch intakten Haut ermöglichen.

In der Pharmazie / Medizin eignet sich so ein mit Wirkstoffen beladenes dreidimensionales Cellulosematerial besonders zur Behandlung in der Wundheilung (z.B. chronische Wunden, Verbrennungen oder diabetische Hautveränderungen) und in der Schmerztherapie oder aber als Einsatz für Implantate.

Pharmazeutisch eingesetzte erfindungsgemäße Zubereitungen enthalten eine sichere und wirksame Menge mindestens eines pharmazeutisch wirksamen Stoffs in den Nanopartikeln. Arzneimittel, die in der Wundbehandlung eingesetzt werden, sollten antiseptische (z.B. Triclosan, Octenidin) oder antibakterielle (z.B. Chloramphenicol, Tetracyclin) Wirkungen zeigen wie auch antimykotisch aktiv sein (z.B. Clotrimazol, Ciclopirox-Olamin). Ebenfalls können Antioxidantien (z.B. Vitamine und Polyphenole) oder die Sauerstoffversorgung verbessernde Wirkstoffe (z.B. Perfluorodekalin) die Wundheilung fördern eingesetzt werden. Weiterhin unterstützen entzündungshemmende und schmerzstillende Wirkstoffe (z.B. Diclofenac, Ibuprofen, Paracetamol) sowie Lokalanästhetika (z.B. Lidocain) die Behandlung. Interessant kann auch der Einsatz von pflanzlichen Wirkstoffen sein, die meist mehrere "heilende" Eigenschaften aufweisen, wie hier nur einige aufgeführte Substanzen: Apigenin, Lutein, Betulin, Weihrauch, Curcumin.

Bei Hautkrankheiten sind wie folgt einige Arzneimittel aufgeführt so gegen Akne (z.B. Isotretinoin, Erythromycin, Isolutrol), gegen Psoriasis (z.B. Calcipotriol, Methoxalen, Dithranol, Immunsuppresiva), gegen Pilzinfektionen (z.B. Terbinafin), gegen Herpesinfektionen (z.B. Aciclovir), gegen allergische Reaktionen (Antihistaminika wie z.B. Dimetinden) und gegen Hautkrebs (z.B. Grüntee, Diclofenac und Psoralene) oder deren photodynamische Therapie (z.B. Temoporfin). Besonders hervorzuheben sind Wirkstoffe gegen Dermatitis und Ekzeme (z.B. Hydrocortison, Prednisolon, Triamcinolonacetonid, Dexamethason, Clobetasol und andere NSAIDs). Ebenfalls können Wirkstoffe (z.B. Peptide, Proteine und Enzyme) eingesetzt werden, die die Zellerneuerung bei Hautveränderungen fördern.

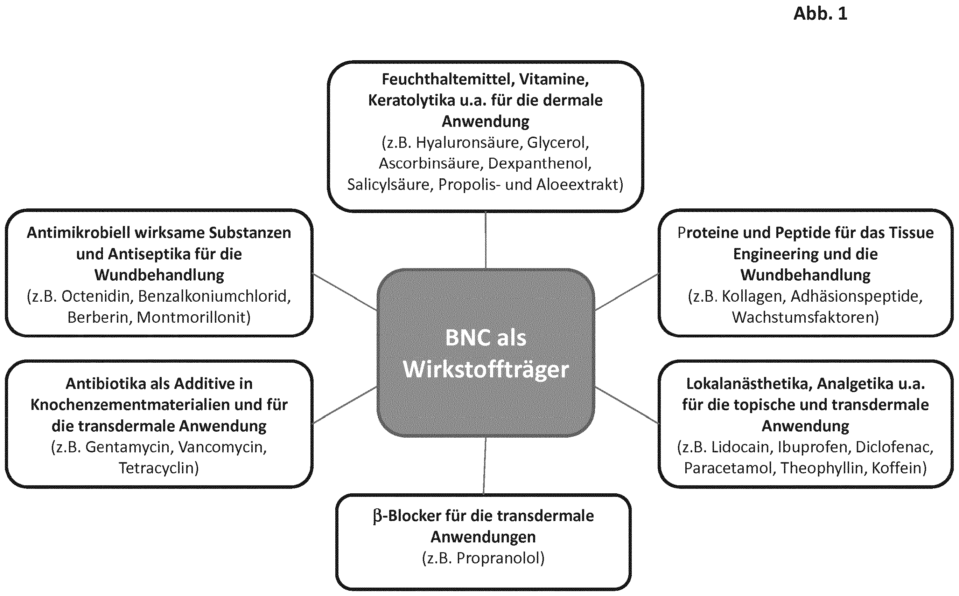

Eine schöne Übersicht der Wirkstoffgruppen, die für den medizinischen Einsatz der BNC als Wirkstoffträger relevant eingestuft werden, ist in der Abb.1 aufgezeigt, entnommen aus der Promotionsarbeit von Astrid Müller (Dissertation 2016, Friedrich-Schiller-Universität, Jena).

Die erfindungsgemäße Zubereitung besonders im pharmazeutischen / medizinischen Bereich für die topische Anwendung kann auch mehrere Nanopartikel unterschiedlicher Art beinhalten. Hier sind nur zwei Beispiele aufgezeigt: erstens, ein nicht-penetrierender Partikel (npP) und ein penetrierender Partikel (pP), was das Einbringen von Wirkstoffen in die unterschiedlichen Haut/Gewebeschichten ermöglicht (npP auf der Hautoberfläche = Stratum corneum und der pP bis in den tieferen Hautareale = z.B. Dermis, Subcutis bis Muskel und Gelenken) und zweitens, ein schnell den Wirkstoff freisetzender Nanopartikel und eine sehr langsame Variante, was eine sofortige als auch eine langandauernde Therapie mit dem Arzneimittel ohne Wechseln der BNC Vliese bewirkt (besonders geeignet für die Schmerz/Wundtherapie).

Im Folgenden sollen die Verfahren zur erfindungsgemäßen Beladung der hydrophilen BNC-Vliese mit den auf Lipiden basierenden Nanopartikeln beschrieben werden. Anhand von einigen Ausführungsbeispielen, die nicht als einschränkend zu verstehen sind, wird das Einbringen des lipophilen, nicht wasserlöslichen Wirkstoffs Coenzym Q10 (Ubiquinon-10) in das dreidimensionale Cellulose-Netzwerk über die Verkapselung in Nanopartikel demonstriert.

Ubiquinon-10 ist eine orange-rote Substanz, die im Körper in den Mitochondrien vorkommt, Bestandteil der Atmungskette ist und somit der Energieerzeugung beiträgt. Aus diesem Grund wird Q10 z.B. in der Kosmetik als Antioxidants und als Anti-Aging Mittel eingesetzt.

Coenzym Q10 ist in höheren Konzentrationen gut in Ethanol und/oder pflanzlichen Ölen löslich und wird so in den kosmetischen Formulierungen eingearbeitet. Eine Beladung der BNC mit einer rein alkoholischen Wirkstofflösung kann zu einer Veränderung der strukturellen und morphologischen Beschaffenheit des dreidimensionalen Cellulose-Netzwerks sowie zu Rekristallisationen der Substanz im Netzwerk führen. Ebenso wird eine homogene Beladung mit einem in "Wasser schwimmenden Q10-Öltröpfchen" nicht durchführbar sein.

Für die Beladung der BNC wurden folgende drei Nanopartikellösungen/Dispersionen ausgewählt, deren Penetrationseigenschaften in die verschiedenen Hautschichten wie auch in tiefer gelegene Gewebeeinheiten beschrieben sind und somit eine erhöhte Bioverfügbarkeit des Arzneimittels am Zielort ermöglichen können: Lipo-Tops, Hydro-Tops und flexible Liposomen (Tabelle 3).

### Ausführungsbeispiele

### 1) BNC:

Die Biosynthese der hier eingesetzten BNC-Vliese erfolgte durch das gramnegative obligat aerobe und nicht pathogene Bakterium *Komagataeibacter xylinus* DSM 14666 (verfügbar bei der Deutschen Sammlung für Mikroorganismen und Zellkulturen, DSMZ, Braunschweig) in einem Hestrin-Schramm Medium bei 28°C in 24-Wells-Platten (Greiner-Bio-one). Genaue Beschreibungen der Kultivierung der Bakterien für die Synthese der BNC, der anschließenden Reinigung der entnommenen Vliese und deren Sterilisation sowie der Gesamtaufbau (Durchmesser, Höhe und Volumen, sowie Gewicht) eines solchen dreidimensionalen Cellulose-Netzwerk werden ausführlich in dem Artikel von Astrid Müller und Kollegen aufgeführt (A. Müller et al.; J.Pharm.Sci 102 (2013) 579-592).

Für die Ausführungen der Beladung der BNC-Vliese wurde zu einem das native, never dried Cellulosematerial sowie zum anderen gefriergetrocknete, lyophylisierte BNC eingesetzt.

### 2) Nanopartikeldispersion und alkoholische Lösung:

Lipo-Tops: bestehend aus Wasser, Soja-Öl, Ethanol, Glyceryl Citrate/Lactate/Linoleate/Oleate, Diglycerylmonooleate und **Q10 (0,5%);** Teilchengröße = 155 nm; PDI = 0,105
Hydro-Tops: bestehend aus Wasser, Glyceryl Citrate/Lactate/Linoleate/Oleate, Ethanol, Ethyloleate, und **Q10 (0,5%);** Teilchengröße = 106 nm; PDI = 0,124
Liposomen: bestehend aus PBS (pH 6,5), Soja-Lecithin (PC 75%), Propylenglycol und **Q10 (0,5%)**
Teilchengröße = 121 nm; PDI = 0,227
Alkoholische Lösung: bestehend aus Ethanol (96%) und Q10 (0,5%)

### 3) Beladung der BNC mit der Nanopartikeldispersion

Beladen vollständig gefriergetrockneter Cellulose-Vliese durch **"Requellen"**

Die gefriergetrockneten BNC-Vliese wurden in einer 24-Wells Platte verteilt. Anschließend wurde langsam und vorsichtig die Menge an Q10-Nanopartikel|ösung über den Querschnitt der BNC verteilt, die dem Verlust an Wasser durch die Gefriertrocknung entspricht.

Beladen nativer "feuchter" Cellulose-Vliese mittels Sorptionsmethode **("Schüttelmethode")**

Die native BNC wurde über eine Dauer von 48 Stunden bei Raumtemperatur unter leichtem Schütteln (70 rpm) mit dem zehnfachen Überschuss (ihres Wassergehalts) mit der Q10-Nanopartikellösung für die Sorption des Wirkstoffs versetzt.

Beladen nativer Cellulose-Vliese mittels Sorptionsmethode (Vortexmethode = **"high speed")**

Die native BNC wurde über eine Dauer von 10 Minuten unter submersen Bedingungen mittels Vortexen (Level 8-9, Vortex Genie 2) mit der wirkstoffhaltigen Nanopartikellösung bei Raumtemperatur beladen (in diesem Fall ebenfalls im zehnfachen Überschuss).

Beladen nativer Cellulose-Vliese mittels **"Kernbeladung"**

Die nativen BNC Vliese wurden in einer 24-Wells Platte verteilt. Danach wurde vorsichtig mit einer Spritze die Q10-Nanopartikeldispersion in den Mittelpunkt der Vliese eingebracht. Die Menge an Wirkstofflösung betrug ein Zehntel der berechneten normalen Wassermenge der BNC.

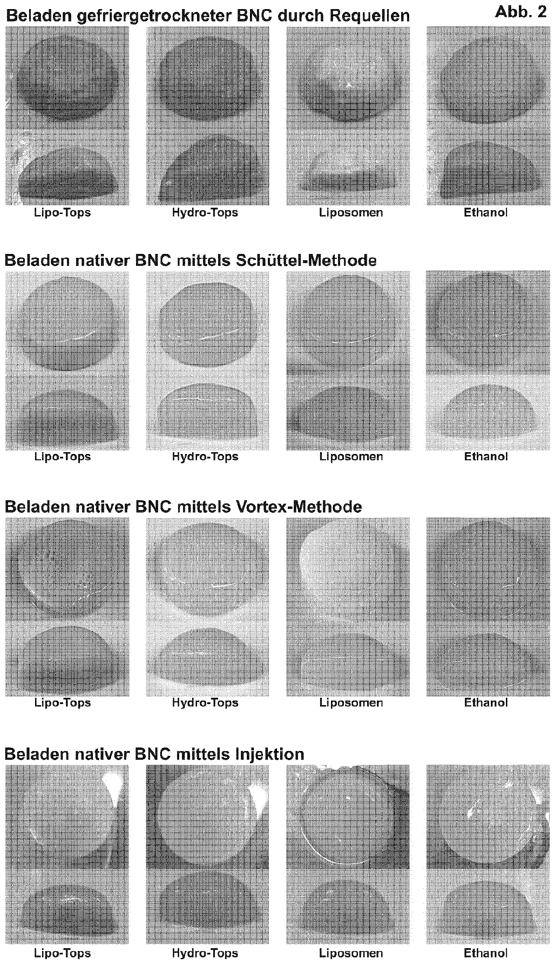

Die Beladungen der BNC mit den unterschiedlichen Formulierungen von Coenzym Q10 entweder in den Trägersystemen verkapselt oder als alkoholische Lösung sind in Abbildung 2 zusammengefasst.

Die Beladungseffizienzen der BNC mit Q10 war vergleichbar, unabhängig von der Art der verwendeten Nanopartikel. So entspricht die Beladungsmenge an Coenzym Q10 in der gefriergetrockneten BNC einheitlich 100% (entspricht der gesamten eingebrachten Nanopartikellösung) und bei der nativen BNC wurden mit allen drei Trägersystemen ca. 10% der als Überschuss eingesetzten Dispersion wiedergefunden.

In das dreidimensionale "never dried" Netzwerk konnte mit den Q10 Nanopartikeln 35% mehr an Q10 eingebracht werden verglichen zu der gefriergetrockneten Variante. Dieser Effekt wird mit der aufgetretenen Strukturveränderung der BNC (Dehydratisierung) durch die Gefriertrocknung begründet (A. Müller et al.; J. Pharmaceutical Sci. 102 (2013) 579-592).

Dagegen sind Unterschiede in der Homogenität der Verteilung des Wirkstoffs durch die verschiedenen Nanopartikel deutlich sichtbar. Eine homogene Verteilung der Q10-Nanopartikel über die gesamte native als auch gefriergetrocknete BNC ist ausschließlich bei einer Beladung mit den Hydro-Tops (W/O/W Emulsion) und den Nano-Tops (O/W Emulsion) nachweisbar. Die Unterschiede in der Färbung der mit den Q10-Tops beladenen Cellulose Vliesen lassen sich mit der unterschiedlichen Art der Nanoemulsionen erklären (O/W versus W/O/W).

Dagegen weisen die Liposomen eine Wirkstoffbeladung fast ausschließlich in den oberen Auftragsflächen auf, d.h. bei der gefriergetrockneten BNC in der weit verzweigten Unterschicht und bei der nativen BNC in der gesamten Oberfläche der Cellulose-Vliese (ausgenommen das Innere der BNC). Erklärbar ist das mit der eigenen hohen, festen Wasserbindung im Bereich der liposomalen Kopfgruppen (bis zu 20 Molekülen Wasser pro Kopfgruppe), eine extrem hohe Wasserummantelung der Vesikel. Erst bei einem Austrocknen der Vesikeloberfläche, wird das Wasser der BNC das "Eindringen" der Liposomen in das Cellulosematerial forcieren (G. Cevc und G. Blume; Biochimica Biophysica Acta 1104 (1992) 226-232).

### 4. Freisetzung des Wirkstoffes aus der BNC

Die Freisetzung von Q10 aus der beladenen BNC wurde über einen Zeitraum von 48 Stunden bei Raumtemperatur mit Hilfe der Franz-Diffusionszelle durchgeführt. Als Akzeptormedium wurde hier reines Wasser eingesetzt, indem das lipophile Q10 als alleiniger purer Wirkstoff nicht löslich ist. Dagegen lässt sich der weiterhin in den Nanopartikeln eingeschlossene Wirkstoff in der wässerigen Lösung gut nachweisen (HPLC).

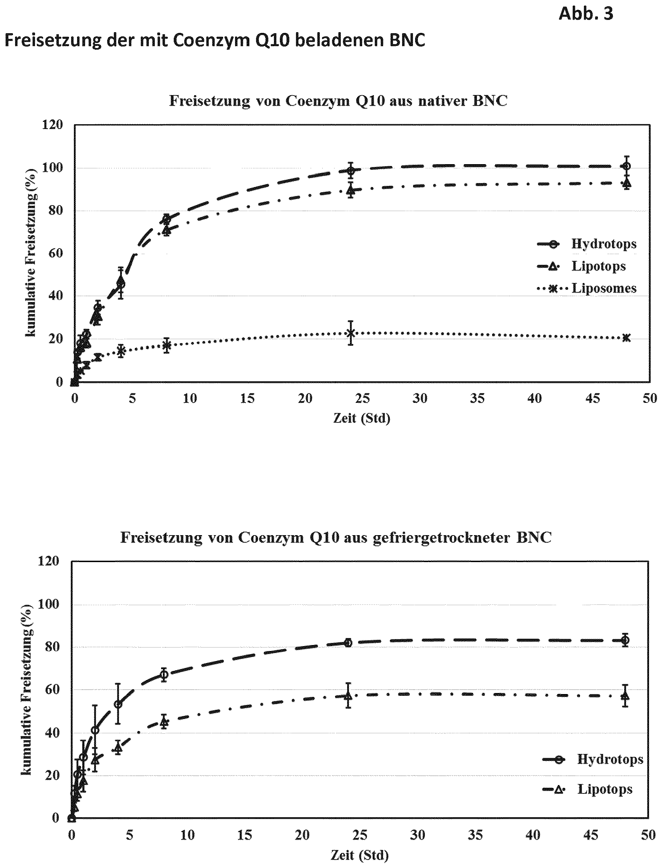

Die Kinetiken der Freisetzung von Coenzym Q10 aus der nativen BNC (" Schüttelmethode") bzw. gefriergetrockneten BNC in Abhängigkeit ihrer Verkapselung in den unterschiedlichen Nanopartikel sind in Abbildung 3 wiedergegeben (Freisetzung der Q10-Liposomen aus der gefriergetrockneten BNC wurde nicht bestimmt, da schon eine geringe Freisetzung aus der BNC bestimmt wurde).

Bei der nativen BNC wurde sowohl für die Hydro-Tops (100%) als auch für die Lipo-Tops (93%) eine vollständige Freisetzung der Nanopartikel mit dem lipophilen Wirkstoff innerhalb von 2 Tagen nachgewiesen. Es wurde zunächst eine schnelle Freisetzung von bis zu 50% der Q10-Tops in den ersten fünf Stunden bestimmt und eine anschließende langsame zweite Phase.

Mit der mit Q10-Liposomen beladenen nativen BNC konnte nur eine unzureichende und langsame Freisetzung erzielt werden, welches wieder auf den eigenen sehr hydrophilen Eigenschaften der Liposomen beruhen wird. Ein Trocknen der liposomalen Vesikeloberfläche erzeugt erst den Drang in eine andere ausgleichende wässerige Umgebungen (Haut) zu diffundieren, was hier nicht gegeben ist (G. Cevc und G. Blume; Biochimica Biophysica Acta 1104 (1992) 226-232). Umgekehrt kann erst eine Freisetzung der Q10-Liposomen erfolgen, wenn die BNC "ausgetrocknet" ist und die Liposomen einen anderen "Wasserlieferanten" aufspüren.

Alle im Akzeptorfluid wiedergefundenen Nanopartikel weisen dieselbe Teilchengröße und Vesikelhomogenität auf wie vor der Beladung der Cellulose. Damit können sie voraussichtlich auch nach ihrer Freisetzung aus der BNC den Wirkstoff in die tieferen Hautschichten transportieren.

Zusammenfassend lassen sich ganz unterschiedliche Produkte des Cellulosematerials für den kosmetischen oder medizinischen Bereich als Wirkstoffträger von schwer wasserlöslichen oder lipophilen Substanzen je nach gewünschter Anwendung (Beladungseffizienz, Freisetzungskinetik und Penetrationsverhalten) herstellen. Folgende drei Faktoren bestimmen dann die Eigenschaft des "fertigen" Endproduktes: 1. die Art des verwendeten Cellulosematerials (Trocknung und Art der Trocknung oder die native Variante), 2. die Art der Beladungsmethode (Requellen, Schüttelmethode, Vortexmethode ) und 3. die Art der Nanopartikel (z.B. Liposomen, O/W Nanoemulsion oder multiple Nanoemulsion samt ihrer Eigenschaften). Ebenfalls kann natürlich die Wasserphase der Nanopartikeldispersion noch einen weiteren hydrophilen Wirkstoff enthalten und die Beladung unterschiedlicher Substanzen in den penetrierenden Vesikel ermöglichen.

**Tabelle: 1**

| R. Mori et al. Clin. Orthop. Relat.Res 469 (2011) 600-606 |
|---|
| => Gentamycin Sulfate + Vancomycin hydrochloride = Antibiotikum |
| => Gefriergetrocknete BNC |
| => Requellen in Antibiotika-Lösung |

| E. Trovatti et al.; Int. J. Pharm 435 (2012) 83-87 |
|---|
| => Lidocain = Lokalanästhetikum |
| => Wasserentzug 50% |
| => Requellen in Puffer + Glycerin 1% + Lidocain |
| => Ibuprofen = Antiphlogistikum / Analgetikum |
| => Wassergehalt der BNC durch EtOH ersetzen, dann 50% entfernen |
| => Requellen in ethanolischer Ibuprofen-Lösung |

| N. Silva et al.; Cellulose 21 (2014) 665-674 |
|---|
| => Caffeine = Anticellulite |
| => Wasserentzug 60% |
| => Requellen in Puffer +1% Glycerol + 5% Ethanol |

| N. Silva et al.; Carbohydrate Polymers 106 (2014) 264-269 |
|---|
| => Diclofenac = Antiphlogistikum / Analgetikum |
| => Wasserentzug 50-60% |
| => Requellen in Puffer + 5% Glycerin + Diclofenac |

| A. Müller et al.; Carbohydrate Polymers 106 (2014) 410-413 |
|---|
| => BSA = Model-Protein |
| => native BNC in BSA-PBS => vortexen |

| S. Moritz et al.; Int. J. Pharm. 471 (2014) 45-55 |
|---|
| => Octenidine dihydrochloride = Antiseptikum |
| => native BNC in Octenidine Lösung |

| C. Wiegand et al.; J. Mater Sei: Mater Med 26 (2015) 245 |
|---|
| => Polyhexanide (PHMB) + Povidone-Iodine (PI) = Antiseptikum |
| => native BNC in PI oder PHMB Lsg (spezielle Puffer) |

| Y. Numata et al.; Int. J. Pharm 486 (2015) 217-225 |
|---|
| => Retinol + Poly(ethylene oxide)-b-poly(caprolactone) => Mizellen (30-70 nm) |
| => native BNC in wässerige Mizellärer-Lösung |

| M. Peres et al., J. Braz. Chem.Sc 27 (2016) 1949-1959 |
|---|
| => Chloroaluminuim phthalocyanine = CIAIPc (Photosensitizer) |
| => Wasserentzug 50% |
| => CIAIPc in ethanolischer Lösung |

| W. Shao et al.; Carbohydrate Polymers 145 (2016) 114-120 |
|---|
| => Tetracycline hydrochloride (THC) = Antibiotikum |
| => native BNC in wässerige THC-Lsg |

| Y. Pötzinger et al.; Poster auf der DPhG Tagung in München, Oktober 2016 |
|---|
| => BNC als "Gen-aktive Matrix" => Beladung mit DNA |
| => Beladung der nativen BNA mittels Injektionen von DNA-Lösung |

| **Y. Alkhatib et al. Universität Jena - in press** |
|---|
| => Octenidine dihydrochloride + Poloxamer = Mizellen (4-5 nm) |
| => native BNC in Octenidine-Polaxamer-Lösung |

**Tabelle: 2**

| **Gattefosse** | | |
|---|---|---|
| Labrafil M1944 | Oleoyl Macrogol-6 Glycerides | pharm. |
| Labrafil M2125 | Linoleoyl Macrogol-6 Glycerides | pharm. |
| Lauroglycol 90 | Propyleneglycol Monolaurate | pharm. |
| Peceol | Glycerol Monooleate | pharm. |
| Plurol Oleique CC 497 | Polyglyceryl-3-Dioleate | pharm. |
| | | |

| **CRODA** | | |
|---|---|---|
| Span 83 | Sorbitan Sesquioleate | pharm. |
| Span 80 | Sorbitan Monooleate | pharm. |
| Cithrol PG3PR | Polyglyceryl-3-Polyricinoleate | pharm. |
| Brij O2 | PEG-2 Oleyl Ether | pharm. |
| Brij O5 | PEG-5 Oleyl Ether | pharm. |
| Brij O10 | PEG-10 Oleyl Ether | pharm. |
| Brij O20 | PEG-20 Oleyl Ether | pharm. |
| Tween 80 | Polysorbate 80 | pharm. |
| | | |

| **Mitsubishi** | | |
|---|---|---|
| Surfhope D-1803 | Sucrose Stearate (Ester) | pharm. |
| Surfhope D-1616 | Sucrose Palmitate (Ester) | pharm. |
| Surfhope D-1216 | Sucrose Laurate (Ester) | pharm. |
| | | |

| **Cremer Care** / **Sasol** | | |
|---|---|---|
| CremerCOOR PPG C12 | Propylene Glycol Laurate | |
| CremerCOOR PG4 | Polyglyceryl-4 Cocoate | |
| CremerCOOR PG3PR | Polyglyceryl-3-5 Ricinoleic Acid | |
| CremerCOOR GMO 90 | Glyceryl Oleate | |
| Imwitor 375 | Glyceryl Citrate/Lactate/Linoleate/Oleate | |
| Imwitor 600 | Polyglyceryl-3 Polyricinoleate | |
| Imwitor 900K | Glyceryl Monostearate 45-50% | |
| Cosmacol ETLP | Dimyristyl Tatrate | |
| | | |

| **Hydrior** | | |
|---|---|---|
| Hydriol PGMO 4 | Polyglycerin-4 Oleate | |
| | | |

| **Dr. Straetmans** | | |
|---|---|---|
| Dermofeel G5 O | Polyglyceryl-5 Oleate | |
| Dermofeel G5 DO | Polyglyceryl-5 Dioleate | |
| | | |

| **NIKKO** | | |
|---|---|---|
| Nikkol DGMO CV | Diglyceryl Monooleate | |
| | | |

| **Sisterna** | | |
|---|---|---|
| Sisterna SP30 | Sucrose Distearate (Ester) | |
| | | |

| **Evonik** | | |
|---|---|---|
| Tego Care SMO V | Sorbitan Monooleate | |
| Tego Care 450 | Polyglyceryl-3 Methylglucose Distearate | |
| Tego Care PS | Methyl Glukose Sesquistearate | |
| Axol C 62 | Glyceryl Stearate Citrate | |
| Varisoft BT 85 | Behentrimonium Chloride | |
| | | |

| **Harke** | | |
|---|---|---|
| Harke Plus EmulPro S5 | Sucose Fatty acid Ester HLB 5 | |
| Harke Plus EmulPro S9 | Sucose Fatty acid Ester HLB 9 | |
| Harke Plus EmulPro S15 | Sucose Fatty acid Ester HLB 15 | |
| | | |

| **Sonstige** | | |
|---|---|---|
| Connock | Sorbitan Trioleate | |
| Lonza | Sorbitan Tristearate | |
| Kowa | Trehalose Isostearate | |
| Berg+Schmidt | Beta Glucan and Pectin | |
| Azelis | Glyceryl Stearate Citrate | |
| Schill+Seilacher | Oleoyl Sarcosine (Aminosr als Kopfgruppe) | |

**Tabelle: 3**

| |
|---|
| Zusammensetzung der bevorzugten penetrierenden Nanopartikel für das Einbringen lipophiler Wirkstoffe in das dreidimensionale Cellulosenetzwerk |

| **Lipo-Tops (O/W Nanoemulsion)** (G. Blume; IFSCC Conference , Paris 2014) |
|---|
| 3 - 7% Imwitor 375 |
| 1 - 3% Coemulgator (u.a. Diglyceryl monooleate; Glyceryl monooleate; Glyceryl monolinoleate) |
| 5-10% Konservierungsmittel (u.a. Ethanol; Propylenglycol; Pentylenglycol) |
| 10-25% natürliche Öle |
| ad 100% Wasser |
| Teilchengrößen vorwiegend zwischen 100-250 nm, PDI vorwiegend zwischen 0.08 und 0.15 Bei den Lipo-Tops befinden sich die lipophilen Wirkstoffe im Vesikelinneren, dem Öltröpfchen. |

| **Hydro-Tops (W/O/W Nanoemulsion)** (G. Blume; Personal Care, Feb. (2012) 37-40) |
|---|
| 7-14% Imwitor 375 |
| 3 - 6% Emollient (u.a. Ethyloleate; Ethyllinoleate; Ethyl-EPA) |
| 5-15% Konservierungsmittel (u.a. Ethanol; Propylenglycol; Pentylenglycol) ad 100% Wasser |
| Teilchengrößen vorwiegend zwischen 80-200 nm, PDI vorwiegend zwischen 0.10 und 0.17 Bei den Hydro-Tops befinden sich die schwer wasserlöslichen oder lipophilen Wirkstoffe im Vesikelinneren, in einem vom Wasser umgebenden Öltröpfchen |

| **Flexible Liposomen** (G. Blume, SÖFW-Journal 126 (2000) 14-17) |
|---|
| 3-10% Lecithin (Soya; Sonnenblume; Raps; Hühnerei - Gehalt an Phosphatidylcholine > 75%) |
| 0 - 5% Emulgator (u.a. Tween 80, Peg₁₀₀-Monoleate, Polyglycerol-Monooleate; Sucrose-Monoleate) |
| 5-15% Konservierungsmittel (u.a. Ethanol; Propylenglycol; Pentylenglycol) ad 100% Phosphatpuffer (pH 6.5) |
| Teilchengrößen vorwiegend zwischen 80-250 nm, PDI vorwiegend zwischen 0.20 und 0.35 Bei den Liposomen sind die lipophilen Wirkstoffe in den lipophilen Teil des Bilayers eingebettet, der einen wässerigen Innenkern umschließt. |
| Zunächst werden die lipophilen Teile samt dem Konservierungsmittel gut miteinander vermischt bzw. gelöst, bis eine homogene Lösung entsteht. Dann wird unter leichtem Vortexen der wässerige Anteil stufenweise zugefügt und solange durchmischt, bei eine homogene Dispersion entsteht. Die Herstellung der Nanopartikel, erfolgt dann bei der anschließenden Hochdruckhomogenisation. |

## Patentansprüche

1. Verfahren zur Herstellung von einem hydrophilen dreidimensionalen Netzwerk bestehend aus Cellulose-Nanofasern mit einer auf Lipiden basierenden Nanopartikeldispersion, welche mindestens einen lipophilen und/oder schwer wasserlöslichen kosmetischen oder pharmazeutischen Wirkstoff beinhaltet,
wobei das Einbringen der Wirkstoffbeladenen Nanopartikeldispersion
- mittels Requellen des angetrockneten oder vollständig getrockneten cellulosehaltigen Materials in der wässerigen Nanopartikellösung erfolgt, oder
- mittels Inkubation der nativen Cellulose in einem Überschuss der wässerigen Nanopartikellösung unter stetigem Schütteln oder im High Speed-Verfahren, oder
- mittels Einbringen der wässerigen Nanopartikellösung in den Kern des cellulosehaltigen Netzwerks.

2. Verfahren gemäß Anspruch 1, wobei die wässerige auf Lipiden basierende Nanopartikeldispersion mindestens einen oder mehrere der folgenden Nanopartikel/Vesikel beinhaltet: Mizellen oder Liposomen oder Niosomen oder Wasser-in-Öl-in-Wasser- Nanoemulsionen (W/O/W) oder Öl-in-Wasser-Nanoemulsionen (O/W) oder Lipid-Nanopartikel, deren Vesikelgröße unter 500 nm liegt.

3. Verfahren gemäß Anspruch 1 oder 2, wobei die wässerige Phase der auf Lipiden basierende Nanopartikeldispersion noch einen oder mehrere unverkapselte hydrophile Wirkstoffe enthalten kann.

4. Verfahren gemäß Anspruch 1 bis 3, wobei die zum Verkapseln des lipophilen und/oder schwer wasserlöslichen kosmetischen und/oder pharmazeutischen Wirkstoffs hergestellten Nanopartikel auf Emulgatoren basieren, die mindestens eine langkettige Fettsäure beinhalten; bevorzugt ungesättigte Fettsäuren wie Ölsäure, Linolsäure und oder Linolensäure.

5. Verfahren gemäß Anspruch 1 bis 4, wobei das Einbringen der lipophilen und/oder schwer wasserlöslichen Wirkstoffe in das cellulosehaltige Material bevorzugt mittels einer W/O/W oder einer O/W Nanoemulsion erfolgt, welche vorwiegend auf dem membranbildenden Emulgator Glyceryl Citrate/Lactate/Linoleate/Oleate basieren oder auf aus Phospholipiden bestehenden Liposomen beruht.

6. Zusammensetzung aus einem hydrophilen dreidimensionalen Netzwerk bestehend aus Cellulose-Nanofasern mit einer auf Lipiden basierenden Nanopartikeldispersion, welche mindestens einen lipophilen und/oder schwer wasserlöslichen kosmetischen oder pharmazeutischen Wirkstoff beinhaltet.

7. Zusammensetzung nach Anspruch 6, wobei die wässerige auf Lipiden basierende Nanopartikeldispersion mindestens einen oder mehrere der folgenden Nanopartikel/Vesikel beinhaltet: Mizellen oder Liposomen oder Niosomen oder Wasser-in-Öl-in-Wasser- Nanoemulsionen (W/O/W) oder Öl-in-Wasser-Nanoemulsionen (O/W) oder Lipid-Nanopartikel, deren Vesikelgröße unter 500 nm liegt.

8. Zusammensetzung nach Anspruch 6 oder 7, wobei der Wirkstoff eine kosmetisch und/oder dermatologisch wirksame lipophile Substanz ist.

9. Zusammensetzung gemäß Anspruch 6 bis 8, wobei der Wirkstoff ausgewählt ist aus der Gruppe umfassend Algen- und Pflanzenextrakt, Anti-Cellulite wirksame Substanz, Anti-Elastase und Anti-Collagenase wirksamer Stoff, anti-inflammatorisch wirksamer Stoff, Antioxidans, Ceramid, hautberuhigender und hautglättender Stoff, Moisturizer, Flavonoid, Phytosterol, Radikalfänger, Saponin, sauerstoffbindende Substanz, sebumreduzierende Substanz, die Durchblutung und Versorgung der Haut fördernder Stoff, Terpen, Vitamin, lipophiles Peptid, Sauerstoffversorgung verbessernder Stoff, entzündungshemmender und schmerzstillender Stoff und Lokalanästhetikum.

10. Zusammensetzung nach einem der Ansprüche 6 bis 9 zur Verwendung in einer topischen therapeutischen, einer dentalen therapeutischen, einer dermatologischen, pharmazeutischen und/oder medizinischen Behandlung.

11. Nicht-therapeutische Verwendung einer Zusammensetzung nach einem der Ansprüche 6 bis 9 zur Verhinderung der Hautalterung und/oder des Photoageings.

12. Zusammensetzung nach einem der Ansprüche 6 bis 9 zur Verwendung bei der Behandlung von Hautkrankheiten und/oder der Wundheilung, in der Schmerztherapie oder in der Implantologie als Einsatz für Implantate, wobei die Hautkrankheiten ausgewählt sind aus der Gruppe umfassend Akne, Psoriasis, Pilzinfektion, Herpesinfektion, allergische Reaktion, Hautkrebs, Dermatitis und Ekzeme.

13. Zusammensetzung zur Verwendung nach Anspruch 12, wobei die Wundheilung chronische Wunden, Verbrennungen oder diabetische Hautveränderungen umfasst.

## Claims

1. A method for producing a hydrophilic three-dimensional network consisting of cellulose nanofibers with a lipid-based nanoparticle dispersion containing at least one lipophilic and/or hardly water-soluble cosmetic or pharmaceutical agent,
the agent-loaded nanoparticle dispersion being introduced
- by re-soaking the partially dried or fully dried cellulose-containing material in the aqueous nanoparticle solution, or
- by incubating the native cellulose in an excess of the aqueous nanoparticle solution under constant shaking or in a high-speed process, or
- by introducing the aqueous nanoparticle solution into the core of the cellulose-containing network.

2. The method according to claim 1, wherein the aqueous lipid-based nanoparticle dispersion contains at least one or more of the following nanoparticles/vesicles: micelles or liposomes or niosomes or water-in-oil-in-water (W/O/W) nanoemulsions or oil-in-water (O/W) nanoemulsions or lipid nanoparticles whose vesicle size is less than 500 nm.

3. The method according to claim 1 or 2, wherein the aqueous phase of the lipid-based nanoparticle dispersion may still contain one or more non-encapsulated hydrophilic agents.

4. The method according to claims 1 to 3, wherein the nanoparticles produced for encapsulating the lipophilic and/or hardly water-soluble cosmetic and/or pharmaceutical agent are based on emulsifiers containing at least one long-chain fatty acid; preferably unsaturated fatty acids such as oleic acid, linoleic acid and/or linolenic acid.

5. The method according to claims 1 to 4, wherein the lipophilic and/or hardly water-soluble agents are preferably introduced into the cellulose-containing material by means of a W/O/W nanoemulsion or an O/W nanoemulsion mainly based on the membrane-forming emulsifier glyceryl citrate/lactate/linoleate/oleate or on liposomes consisting of phospholipids.

6. A composition composed of a hydrophilic three-dimensional network consisting of cellulose nanofibers with a lipid-based nanoparticle dispersion containing at least one lipophilic and/or hardly water-soluble cosmetic or pharmaceutical agent.

7. The composition according to claim 6, wherein the aqueous lipid-based nanoparticle dispersion contains at least one or more of the following nanoparticles/vesicles: micelles or liposomes or niosomes or water-in-oil-in-water (W/O/W) nanoemulsions or oil-in-water (O/W) nanoemulsions or lipid nanoparticles whose vesicle size is less than 500 nm.

8. The composition according to claim 6 or 7, wherein the agent is a cosmetically and/or dermatologically active lipophilic substance.

9. The composition according to claims 6 to 8, wherein the agent is selected from the group comprising algae and plant extract, anti-cellulite active substance, anti-elastase and anti-collagenase active substance, anti-inflammatory active substance, antioxidant, ceramide, skin-soothing and skin-smoothing substance, moisturizer, flavonoid, phytosterol, radical scavenger, saponin, oxygen-binding substance, sebum-reducing substance, substance promoting the perfusion and supply of the skin, terpene, vitamin, lipophilic peptide, substance improving oxygen supply, anti-inflammatory and analgesic substance, and local anesthetic.

10. The composition according to any one of claims 6 to 9 for use in a topical therapeutic treatment, a dental therapeutic treatment, a dermatological treatment, a pharmaceutical treatment and/or a medical treatment.

11. A non-therapeutic use of a composition according to any one of claims 6 to 9 for preventing skin aging and/or photo-aging.

12. The composition according to any one of claims 6 to 9 for use in treating skin diseases and/or wound healing, in pain therapy or in implantology as an insert for implants, wherein the skin diseases are selected from the group comprising acne, psoriasis, fungal infection, herpes infection, allergic reaction, skin cancer, dermatitis, and eczema.

13. The composition for use according to claim 12, wherein wound healing comprises chronic wounds, burns or diabetic skin lesions.

## Revendications

1. Procédé de préparation d'un réseau tridimensionnel hydrophile se composant de nanofibres de cellulose avec une dispersion de nanoparticules à base de lipides contenant au moins un agent cosmétique ou pharmaceutique lipophile et/ou faiblement soluble dans l'eau,
dans lequel la dispersion de nanoparticules chargée de l'agent est introduite
- en faisant regonfler le matériau contenant de la cellulose et partiellement ou entièrement séché dans la solution de nanoparticules aqueuse ou
- en incubant la cellulose native dans un excès de la solution de nanoparticules aqueuse tout en l'agitant constamment ou dans un procédé à haute vitesse ou
- en introduisant la solution de nanoparticules aqueuse dans le noyau du réseau contenant de la cellulose.

2. Procédé selon la revendication 1, dans lequel la dispersion de nanoparticules aqueuse à base de lipides contient au moins une ou plusieurs des nanoparticules/vésicules suivantes : des micelles ou des liposomes ou des niosomes ou des nanoémulsions eau-dans-huile-dans-eau (W/O/W) ou des nanoémulsions huile-dans-eau (O/W) ou des nanoparticules lipides dont la taille des vésicules est inférieure à 500 nm.

3. Procédé selon la revendication 1 ou 2, dans lequel la phase aqueuse de la dispersion de nanoparticules à base de lipides peut encore contenir un ou plusieurs agents hydrophiles non encapsulés.

4. Procédé selon les revendications 1 à 3, dans lequel les nanoparticules produites pour l'encapsulation de l'agent cosmétique et/ou pharmaceutique lipophile et/ou faiblement soluble dans l'eau sont à base d'émulsifiants contenant au moins un acide gras à longue chaîne ; de préférence des acides gras insaturés tels que l'acide oléique, l'acide linoléique et/ou l'acide linolénique.

5. Procédé selon les revendications 1 à 4, dans lequel les agents lipophiles et/ou faiblement solubles dans l'eau sont de préférence introduits dans le matériau contenant de la cellulose au moyen d'une nanoémulsion W/O/W ou d'une nanoémulsion O/W principalement à base de l'émulsifiant de formation de membrane glycéryle citrate/lactate/linoléate/oléate ou à base de liposomes se composant de phospholipides.

6. Composition composée d'un réseau tridimensionnel hydrophile se composant de nanofibres de cellulose avec une dispersion de nanoparticules à base de lipides contenant au moins un agent cosmétique ou pharmaceutique lipophile et/ou faiblement soluble dans l'eau.

7. Composition selon la revendication 6, dans laquelle la dispersion de nanoparticules aqueuse à base de lipides contient au moins une ou plusieurs des nanoparticules/vésicules suivantes: des micelles ou des liposomes ou des niosomes ou des nanoémulsions eau-dans-huile-dans-eau (W/O/W) ou des nanoémulsions huile-dans-eau (O/W) ou des nanoparticules lipides dont la taille des vésicules est inférieure à 500 nm.

8. Composition selon la revendication 6 ou 7, dans laquelle l'agent est une substance lipophile ayant de l'activité cosmétique et/ou dermatologique.

9. Composition selon les revendications 6 à 8, dans laquelle l'agent est choisi dans le groupe comprenant de l'extrait d'algues ou de plante, de la substance ayant une activité anti-cellulite, de la substance ayant une activité anti-élastase et anti-collagénase, de la substance ayant une activité anti-inflammatoire, de l'antioxydant, du céramide, de la substance apaisante et lissante pour la peau, de l'hydratant, du flavonoïde, du phytostérol, du capteur de radicaux, du saponine, de la substance adsorbant l'oxygène, de la substance réduisant le sébum, de la substance promouvant la perfusion et l'alimentation de la peau, du terpène, de la vitamine, du peptide lipophile, de la substance améliorant l'alimentation en oxygène, de la substance anti-inflammatoire et analgésique et de l'anesthésique local.

10. Composition selon l'une quelconque des revendications 6 à 9 pour l'utilisation dans un traitement thérapeutique topique, thérapeutique dentaire, dermatologique, pharmaceutique et/ou médical.

11. Utilisation non thérapeutique d'une composition selon l'une quelconque des revendications 6 à 9 pour la prévention du vieillissement de la peau et/ou du photo-vieillissement.

12. Composition selon l'une quelconque des revendications 6 à 9 pour l'utilisation dans le traitement de maladies de la peau et/ou la guérison de blessures, dans le traitement de la douleur ou dans l'implantologie comme insert pour des implants, dans laquelle les maladies de la peau sont choisies dans le groupe comprenant l'acné, le psoriasis, l'infection fongique, l'infection herpétique, la réaction allergique, le cancer de la peau, la dermatite et l'eczéma.

13. Composition pour l'utilisation selon la revendication 12, dans laquelle la guérison de blessures comprend des blessures chroniques, des brûlures ou des lésions cutanées diabétiques.
